# EUROPÄISCHE PATENTSCHRIFT

(11) **EP 2 438 889 B1**
(45) Veröffentlichungstag und Bekanntmachung des Hinweises auf die Patenterteilung: **11.12.2013**
(21) Anmeldenummer: 10186622.6
(22) Anmeldetag: 05.10.2010
(51) Int. Cl.: A61F 2/38, A61F 2/00

(54) **Kniegelenkprothese**
Knee joint prosthesis
Prothèse d'articulation du genou

(43) Veröffentlichungstag der Anmeldung: 11.04.2012
(73) Patentinhaber: AESCULAP AG, 78532 Tuttlingen (DE)
(72) Erfinder: Mouillet, Dominique, 52000, Semoutiers-Montsaon (FR); Idler, Uwe, 78576 Liptingen (DE); Moussa, Saïd, 52000, Chamarandes-Choignes (FR)
(74) Vertreter: Hoeger, Stellrecht & Partner Patentanwälte

(56) Entgegenhaltungen:
- EP-A1- 2 145 606
- EP-A2- 1 591 082
- US-A- 6 013 103
- US-A1- 2010 161 067

## Beschreibung

Die vorliegende Erfindung betrifft eine Kniegelenkendoprothese mit einer Femurkomponente und einer Tibiakomponente, welche zusammenwirkende und miteinander in Kontakt stehende Gelenkflächen umfassen, wobei ferner eine Rotationsführungseinrichtung vorgesehen ist zum Führen einer Zwangsrotation der Femurkomponente und der Tibiakomponente relativ zueinander um ein mediales Rotationszentrum infolge einer Flexion der Kniegelenkendoprothese, welche Rotationsführungseinrichtung ein erstes Führungselement mit einer ersten Führungsfläche und ein zweites Führungselement mit einer zweiten, mit der ersten Führungsfläche zusammenwirkenden Führungsfläche umfasst, wobei die Tibiakomponente das erste Führungselement und die Femurkomponente das zweite Führungselement umfassen.

In den letzten Jahren wurde bei der Konstruktion von Kniegelenkendoprothesen zunehmend Wert darauf gelegt, die natürliche Kniekinematik bestmöglich nachzubilden. Im natürlichen Knie vollführt der Femur eine Roll-Gleit-Bewegung auf der Tibia. Dieser sogenannten "Roll-Back"-Bewegung des Femurs ist im natürlichen Knie ferner eine Rotationsbewegung um die Längsachse im medialen Bereich des Knies, das auch als mediales Kompartment bezeichnet wird, überlagert. Diese mediale Rotationsbewegung wird auch "Medial-Pivot"-Bewegung genannt. Dies bedeutet, dass der mediale Teil während eines Gangzyklus relativ zur Tibia nahezu stillsteht, also im Wesentlichen ein Drehpunkt oder Rotationszentrum medialseitig definiert wird. Dagegen vollführt der laterale Teil des Femurs relativ zur Tibia eine Art "bananenförmige" Bewegung nach dorsal. Insgesamt ergibt sich somit als Gesamtkinematik des natürlichen Kniegelenkes während eines normalen Gangzyklus eine Rotationsbewegung des Femur um die mediale Gelenkfläche der Tibiakomponente.

Eine Kniegelenkendoprothese mit unsymmetrischen Gelenkflächen der Tibiakomponente ist beispielsweise aus der EP 0 927 009 A1 bekannt. Allerdings ist bei dieser Kniegelenkendoprothese die Rotationsführungseinrichtung gebildet durch die Gelenkflächen der Tibiakomponente, und zwar durch deren besondere Form. Dagegen ist eine Kniegelenkendoprothese der eingangs beschriebenen Art aus der EP 2 145 606 A1 bekannt. Nachteil dieser bekannten Kniegelenkendoprothese ist es jedoch, dass für rechte und linke Kniegelenkendoprothesen jeweils unterschiedliche Femur- und Tibiakomponenten für das linke Knie und das rechte Knie vorgesehen werden müssen.

Eine Kniegelenkendoprothese der eingangs beschriebenen Art ist beispielsweise aus der EP 1 591 082 A2 bekannt, welche die Basis für den Oberbegriff des Anspruchs 1 bildet. Weitere Kniegelenkendoprothesen sind ferner in der US 6,013,103 sowie in der US 2010/0161067 A1 beschrieben.

Es ist daher Aufgabe der vorliegenden Erfindung, eine Kniegelenkendoprothese der eingangs beschriebenen Art so zu verbessern, dass deren Funktion verbessert wird.

Diese Aufgabe wird bei einer Kniegelenkendoprothese der eingangs beschriebenen Art erfindungsgemäß dadurch gelöst, dass die erste Führungsfläche einen ersten Krümmungsradius definiert, dass die zweite Führungsfläche einen zweiten Krümmungsradius definiert, dass der erste und der zweite Krümmungsradius identisch sind, dass die zweite Führungsfläche definiert wird durch einen Ausschnitt einer äußeren Oberfläche eines Gewindes, welches einen ausgerundeten Gewindegrund mit einem Gewindegrundradius aufweist, welcher dem ersten Krümmungsradius entspricht.

Eine derart ausgebildete Kniegelenkendoprothese ermöglicht es insbesondere, die Tibiakomponente vollständig spiegelsymmetrisch zu einer in anterior-posteriorer Richtung verlaufenden Spiegelebene auszubilden. Eine solche Tibiakomponente kann dann optional zur Ausbildung sowohl eines künstlichen linken als auch rechten Kniegelenks genutzt werden. Auf diese Weise kann die Zahl der bereitzuhaltenden Prothesenkomponenten deutlich verringert werden. Insbesondere kann so die Zahl der bereitzuhaltenden Prothesenkomponenten auf die Hälfte reduziert werden. Dadurch können Lagerhaltungskosten für den Hersteller sowie für die Klinik, in der der Austausch eines ganz oder teilweise geschädigten Kniegelenks durch eine Kniegelenkendoprothese vorgenommen wird, deutlich verringert werden. Des Weiteren können auch Verwechslungen bei der Implantation vermieden werden. Dies kommt in der Praxis bedauerlicherweise immer wieder vor, da bei zahlreichen bekannten Kniegelenkendoprothesen die Unterschiede zwischen rechter und linker Tibiakomponente häufig nicht sehr groß und für einen Operateur nur schwer zu erkennen sind. Trotz der deutlich vereinfachten Ausgestaltung der Kniegelenkendoprothese ermöglicht es diese, die natürliche Kniekinematik wie oben beschrieben sehr gut nachzubilden. Insbesondere kann die Rotationsführungseinrichtung zumindest teilweise die Funktion des hinteren Kreuzbands übernehmen, das heißt sie kann zudem auch die Femurkomponente relativ zur Tibiakomponente in Position halten und ein Verrutschen der Femurkomponente relativ zur Tibiakomponente in anteriorer Richtung verhindern. Günstig ist es, dass die zweite Führungsfläche definiert wird durch einen Ausschnitt einer äußeren Oberfläche eines Gewindes, welches einen ausgerundeten Gewindegrund mit einem Gewindegrundradius aufweist, welcher dem ersten Krümmungsradius entspricht. Eine derartige Ausgestaltung der zweiten Führungsfläche ermöglicht insbesondere automatisch eine Transversalbewegung der Femurkomponente relativ zur Tibiakomponente infolge einer Flexionsbewegung zu erzwingen. Eine solche Transversalbewegung ist durch die gewindeförmige Ausgestaltung der zweiten Führungsfläche auf einfache Weise zu realisieren. Insbesondere kann beispielsweise durch die Steigung des Gewindes das Maß für eine Transversalbewegung gezielt vorgegeben werden. Ferner gestattet es diese Ausgestaltung der zweiten Führungsfläche, mit einer ersten, vollständig spiegelsymmetrisch ausgebildeten ersten Führungsfläche zusammenzuwirken und eine linienförmige Anlage zu realisieren. Vorteilhafterweise ist der Gewindegrundradius unabhängig von einer Dreh- oder Rotationsstellung des zweiten Führungselements, wobei die Rotationsstellung einem Flexionswinkels des Knies entspricht.

Die Ausgestaltung der Kniegelenkendoprothese lässt sich insbesondere dadurch vereinfachen, dass der erste und der zweite Krümmungsradius von einem Flexionswinkel der Kniegelenkendoprothese unabhängig sind. Die identischen und konstanten Krümmungsradien der beiden Führungsflächen ermöglichen zudem eine optimale Anlage aneinander und somit im Vergleich zu bekannten Kniegelenkendoprothesen eine deutlich verringerte Flächenpressung im Bereich der aneinander anliegenden Führungselemente der Rotationsführungseinrichtung.

Besonders einfach wird der Aufbau der Kniegelenkendoprothese, wenn die erste Führungsfläche spiegelsymmetrisch zu einer in anterior-posteriorer Richtung verlaufenden ersten Spiegelebene ausgebildet ist. Diese besondere Formgebung der ersten Führungsfläche ermöglicht es insbesondere, die Tibiakomponente insgesamt, also vollständig, spiegelsymmetrisch zur ersten Spiegelebene auszubilden. Dadurch kann die Tibiakomponente zur Ausbildung sowohl einer linken als auch einer rechten Kniegelenkendoprothese genutzt werden.

Besonders einfach wird die Ausgestaltung der Kniegelenkendoprothese, wenn die erste Führungsfläche einen Ausschnitt einer Zylinderoberfläche definiert. Mit anderen Worten kann das erste Führungselement einen Teil eines Zylinders bilden, wobei die erste Führungsfläche einen Teil einer äußeren Oberfläche des zylindrischen Bereichs des ersten Führungselements bilden kann.

Gemäß einer weiteren bevorzugten Ausführungsform der Erfindung kann vorgesehen sein, dass die erste und die zweite Führungsfläche in einem Flexionswinkelbereich, welcher durch einen minimalen und einen maximalen Flexionswinkel definiert wird, längs eines ebenen Kreisbogenabschnitts aneinander anliegen und bei einer Flexionsbewegung aneinander abrollen und/oder aneinander abgleiten. Eine derart ausgebildete Kniegelenkendoprothese ermöglicht nicht nur eine punktförmige Anlage der ersten und zweiten Führungsfläche aneinander, wie dies bei bekannten Prothesen häufig der Fall ist, sondern mindestens eine linienförmige Anlage entlang des ebenen Kreisbogenabschnitts. Dadurch können Kräfte zwischen Femur- und Tibiakomponente deutlich einfacher und sicherer übertragen werden. Bei gleicher Stabilität könnte so insbesondere die Größe der Führungselemente im Vergleich zu herkömmlichen Kniegelenkendoprothesen verkleinert werden. Die Kniegelenkendoprothese kann insbesondere so ausgebildet sein, dass die ersten und zweiten Führungsflächen bei einer Flexionsbewegung des Knies aneinander abrollen. Sie können aber auch alternativ aneinander abgleiten oder eine kombinierte Gleit-AbrollBewegung relativ zueinander ausführen.

Weiter vereinfachen lässt sich der Aufbau der Kniegelenkendoprothese, wenn ein Radius des Kreisbogenabschnitts dem ersten und zweiten Krümmungsradius entspricht. Insbesondere kann dies erreicht werden, wenn das zweite Führungselement senkrecht auf die erste Führungsfläche einwirkt.

Günstig ist es ferner, wenn die Rotationsführungseinrichtung eine Rotationsachse einer Rotationsbewegung der Femurkomponente und der Tibiakomponente relativ zueinander definiert und wenn die Rotationsachse parallel zu einer Schnittlinie zwischen der ersten Führungsfläche und der ersten Spiegelebene verläuft. Diese so definierte Rotationsachse muss nicht zwingend mit der oben beschriebenen medialen Rotationsachse beziehungsweise dem medialen Rotationszentrum zwischen Femur- und Tibiakomponente zusammenfallen. Die definierte Rotationsachse ergibt sich aufgrund der zueinander korrespondierenden Ausbildung der ersten und zweiten Führungsflächen. Eine überlagerte Rotationsbewegung von Femur- und Tibiakomponente um ein mediales Rotationszentrum ergibt sich aufgrund der jeweiligen Ausgestaltung der aneinander anliegenden Gelenkflächen unter Berücksichtigung der überlagerten Gleit-Abroll-Bewegung der aneinander anliegenden Gelenkflächen von Femurund Tibiakomponente. Mit anderen Worten wird die oben beschriebene Rotationsachse definiert durch die zusammenwirkenden ersten und zweiten Führungsflächen.

Der Aufbau der Kniegelenkendoprothese kann weiter vereinfacht werden, wenn der Kreisbogenabschnitt eine Schnittebene definiert, welche senkrecht zur Schnittlinie verläuft. Damit ist zudem sichergestellt, dass die zweite Führungsfläche stets senkrecht auf die erste Führungsfläche einwirkt.

Grundsätzlich wäre es zwar denkbar, dass die Schnittlinie senkrecht oder im Wesentlichen senkrecht auf einer von der Tibiakomponente definierten Tibiaebene steht. Vorteilhaft ist es jedoch, wenn die Schnittlinie relativ zu einer Flächennormalen einer von der Tibiakomponente definierten Tibiaebene geneigt ist. Vorzugsweise ist ein freies Ende des ersten Führungselements etwas in posteriorer Richtung geneigt. Auf diese Weise lässt sich eine Bewegung der Femurkomponente relativ zur Tibiakomponente besonders gut führen und zudem eine Luxation der Kniegelenkendoprothese auf einfache Weise nahezu sicher verhindern.

Vorteilhaft ist es ferner, wenn die Femurkomponente zwei Gelenkflächen umfasst, welche spiegelsymmetrisch zu einer im Wesentlichen in anterior-posteriorer Richtung verlaufenden zweiten Spiegelebene ausgebildet sind. Dies ermöglicht es insbesondere, sowohl die Gelenkflächen der Femurkomponente als auch die Gelenkflächen der Tibiakomponente jeweils spiegelsymmetrisch zueinander auszubilden. Eine Unsymmetrie der Kniegelenkendoprothese ist daher allenfalls für die zweite Führungsfläche und damit für das zweite Führungselement erforderlich, um eine Kniegelenkendoprothese mit einer möglichst natürlichen Kinematik auszubilden.

Günstig kann es ferner sein, wenn die zweite Führungsfläche derart ausgebildet ist, dass bei einer Flexion der Kniegelenkendoprothese mit einem Flexionswinkel im Flexionswinkelbereich der Kreisbogenabschnitt für jeden Flexionswinkel einen Mittelpunkt definiert, und wenn die Mittelpunkte auf einer Mittelpunktsebene liegen, welche parallel zur zweiten Spiegelebene in lateraler Richtung versetzt verläuft. Durch diese besondere Ausgestaltung der zweiten Führungsfläche kann eine Querbewegung der Femurkomponente relativ zur Tibiakomponente etwas in medialer Richtung vorgegeben werden infolge einer Beugungsbewegung des Knies.

Vorteilhafterweise nimmt ein Abstand der Mittelpunktsebene von der zweiten Spiegelebene in Abhängigkeit des Flexionswinkels zu. Damit ist es insbesondere möglich, die Femurkomponente relativ zur Tibiakomponente um ein mediales Rotationszentrum zu rotieren infolge einer Flexionsbewegung.

Grundsätzlich wäre es denkbar, dass ein Abstand der Mittelpunktsebene von der zweiten Spiegelebene in Abhängigkeit des Flexionswinkels linear zunimmt. Besonders günstig ist es jedoch, wenn ein Abstand der Mittelpunktsebene von der zweiten Spiegelebene in Abhängigkeit des Flexionswinkels nichtlinear zunimmt. Eine Rotationsführungseinrichtung mit einer derartigen Abhängigkeit des Abstands der zweiten Spiegelebene von der Mittelpunktsebene gestattet es, eine Kinematik des natürlichen Knies nahezu perfekt nachzubilden.

Vorzugsweise sind die zwei Gelenkflächen der Femurkomponente in Form von spiegelsymmetrisch zur zweiten Spiegelebene angeordneter Kondylenflächen ausgebildet. Eine solche Femurkomponente lässt sich besonders einfach und präzise herstellen.

Günstigerweise sind in einer Extensionsstellung der Kniegelenkendoprothese die erste Spiegelebene und die zweite Spiegelebene identisch. Mit anderen Worten fallen die erste und die zweite Spiegelebene zusammen, wenn das Knie gestreckt ist, also ein Beugungs- oder Flexionswinkel zwischen Femurund Tibiakomponente 0° beträgt.

Prinzipiell wäre es denkbar, die Tibiakomponente einteilig auszubilden. Vorteilhaft ist es jedoch, wenn die Tibiakomponente ein Tibiateil und ein am Tibiateil gelagertes Meniskusteil umfasst und wenn das Meniskusteil mit den Gelenkflächen der Femurkomponente zusammenwirkende Gelenkflächen umfasst. Eine solche mindestens zweiteilige Ausgestaltung der Tibiakomponente ermöglicht es insbesondere, die Tibiakomponente aus unterschiedlichen Materialien herzustellen. Beispielsweise kann zur Ausbildung hochgleitfähiger Gelenkflächen das Meniskusteil aus einem Kunststoff, zum Beispiel aus Polyethylen mit einem hohen Molekulargewicht hergestellt werden. Das Tibiateil, um eine besonders hohe Stabilität insbesondere für die Verankerung desselben an der Tibia zu erreichen, kann beispielsweise aus einem hochfesten metallischen Werkstoff hergestellt werden. Insbesondere kann es sich dabei um eine Kobalt-Chrom-Basislegierung handeln.

Grundsätzlich wäre es denkbar, das erste Führungselement am Tibiateil auszubilden. Der Aufbau der Kniegelenkendoprothese lässt sich jedoch deutlich vereinfachen, wenn das Meniskusteil das erste Führungselement umfasst. Insbesondere kann das erste Führungselement in Form eines abstehenden Vorsprungs ausgebildet sein. Ferner kann das Tibiateil beispielsweise eine ein Tibiaplateau definierende Tibiaplatte aufweisen, auf welcher das Meniskusteil großflächig anliegt. Zum Beispiel kann es dort mittels einer Schraube oder eines anderen bekannten Verbindungsmechanismus befestigt werden, um im Revisionsfall ein Lösen des Meniskusteils vom Tibiateil zu gestatten.

Vorteilhaft ist es, wenn das Meniskusteil und/oder das Tibiateil spiegelsymmetrisch zu einer in anterior-posteriorer Richtung verlaufenden ersten Spiegelebene ausgebildet sind. Dies bedeutet, dass wahlweise das Meniskusteil oder das Tibiateil spiegelsymmetrisch zur ersten Spiegelebene ausgebildet sein können. Optional können auch beide Teile spiegelsymmetrisch ausgebildet sein. Denkbar ist es insbesondere, das Tibiateil nicht spiegelsymmetrisch auszubilden. Dies kann insbesondere vorteilhaft sein, wenn das Tibiateil modular ausgebildet ist, um eine individuell optimierte Verankerung beispielsweise eines Schafts des Tibiateils an der verbliebenen Tibia des Patienten zu erreichen.

Günstig ist es ferner, wenn die zweite Spiegelebene und die erste Spiegelebene identisch sind für Flexionswinkel zwischen Tibiakomponente und Femurkomponente, welche kleiner sind als ein Grenzwinkel. Mit anderen Worten bedeutet dies, dass Tibia und Femur relativ zueinander gebeugt werden können in einem Winkelbereich ausgehend von einer Streckstellung bis zum definierten Grenzwinkel, wobei ausschließlich eine Beugung erfolgt, jedoch noch keine Rotation der Femurkomponente relativ zur Tibiakomponente um ein mediales Rotationszentrum.

Günstig ist es, wenn der Grenzwinkel dem minimalen Flexionswinkel des Flexionswinkelbereichs entspricht. Dies ermöglicht insbesondere eine Ausbildung der Kniegelenkendoprothese derart, dass die erste und die zweite Führungsfläche erst ab einem Flexionswinkel, der dem Grenzwinkel entspricht, miteinander in Kontakt stehen, für kleinere Flexionswinkel dagegen nicht.

Vorteilhafterweise weist der erste Krümmungsradius einen Wert auf, welcher in einem Bereich von etwa 7 mm bis etwa 15 mm liegt. Eine erste Führungsfläche mit einem derartigen Krümmungsradius auszubilden ermöglicht es, ein ausreichend stabiles Führungselement vorzusehen, sei es am Meniskusteil oder auch am Tibiateil.

Gemäß einer bevorzugten Ausführungsform der Erfindung beträgt der erste Krümmungsradius 12 mm.

Besonders günstig ist es, wenn das Gewinde eine nichtlineare Steigung aufweist. Dies ermöglicht es, eine Transversalbewegung in einer Form zu realisieren, welche einer solchen Bewegung beim natürlichen Knie nachempfunden ist.

Günstigerweise nimmt die Steigung des Gewindes in Abhängigkeit des Flexionswinkels zu. Auf diese Weise lässt sich mit der Rotationsführungseinrichtung eine dem natürlichen Knie bestmöglich nachempfundene Relativbewegung zwischen Femurkomponente und Tibiakomponente erzwingen.

Eine optimale Krafteinleitung vom ersten Führungselement auf das zweite Führungselement und auch umgekehrt lässt sich insbesondere dadurch erreichen, dass das zweite Führungselement in einem Bereich zwischen den Gelenkflächen der Femurkomponente ausgebildet ist.

Die Funktion des hinteren Kreuzbands lässt sich durch die Rotationsführungseinrichtung insbesondere auf einfache Weise dadurch übernehmen, dass das zweite Führungselement in einem posterioren Endbereich der Femurkomponente ausgebildet ist.

Günstig ist es, wenn die Femurkomponente einstückig ausgebildet ist. Dadurch lässt sich deren Stabilität erhöhen und auch eine Verankerung am verbliebenen Femur vereinfachen.

Vorteilhaft kann es ferner sein, wenn das Tibiateil und/oder das Meniskusteil einstückig ausgebildet sind. Auch hier lässt sich eine Stabilität der jeweiligen Teile bei einer einstückigen Ausbildung verbessern. Allerdings kann es auch vorteilhaft sein, wenn das Tibiateil in Form eines modular ausgebildeten Tibiateils gestaltet ist. Es lässt sich dann individuell an die Physiologie eines Patienten anpassen, um eine optimale Verankerung an der verbliebenen Tibia zu erreichen.

Günstig ist es, wenn die Tibiakomponente einstückig ausgebildet ist. Dies ermöglicht es insbesondere, das Tibiateil und das Meniskusteil einstückig miteinander auszubilden. Ferner eröffnet diese Ausgestaltung die Möglichkeit, die Tibiakomponente komplett aus einem Kunststoff herzustellen. Beispielsweise kann als Werkstoff für eine solche einstückige Tibiakomponente Polyethylen gewählt werden, vorzugsweise Polyethylen mit hohem Molekulargewicht (HMW-PE).

Die nachfolgende Beschreibung bevorzugter Ausführungsformen der Erfindung dient im Zusammenhang mit den Zeichnungen der näheren Erläuterung. Es zeigen:
- Figur 1a:: eine schematische perspektivische Gesamtansicht einer implantierten Kniegelenkendoprothese bei schwacher Beugung;
- Figur 1b:: eine schematische perspektivische Gesamtansicht einer Kniege-lenkendoprothese aus Figur 1 bei stärkerer Beugung;

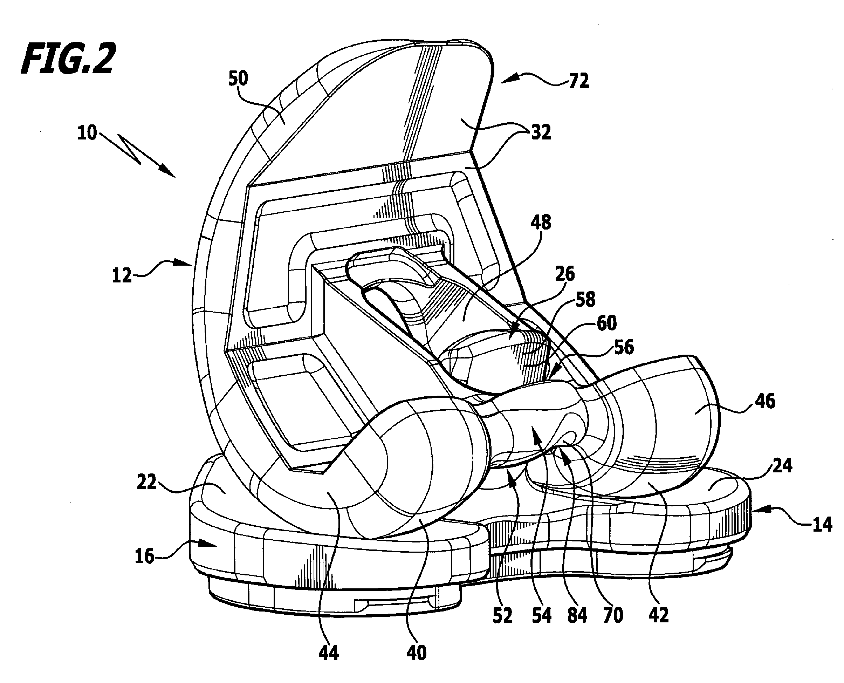

- Figur 2:: eine perspektivische Gesamtansicht der Kniegelenkendoprothese aus Figur 1a in einer Beugestellung von etwa 45°;

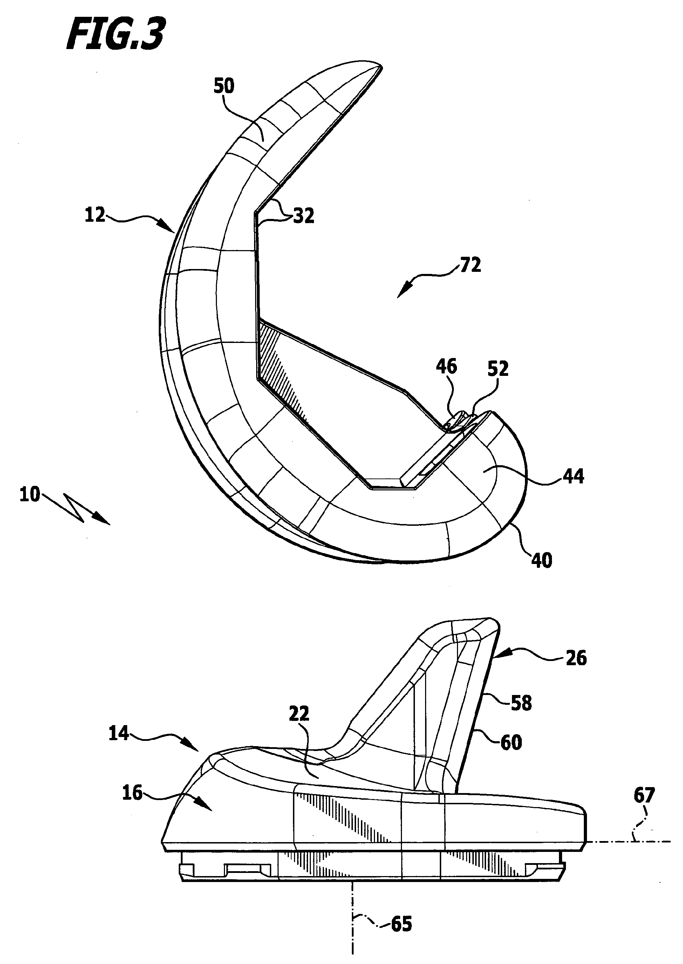

- Figur 3:: eine Explosionsdarstellung der Kniegelenkendoprothese aus Figur 2 in einer Seitenansicht;

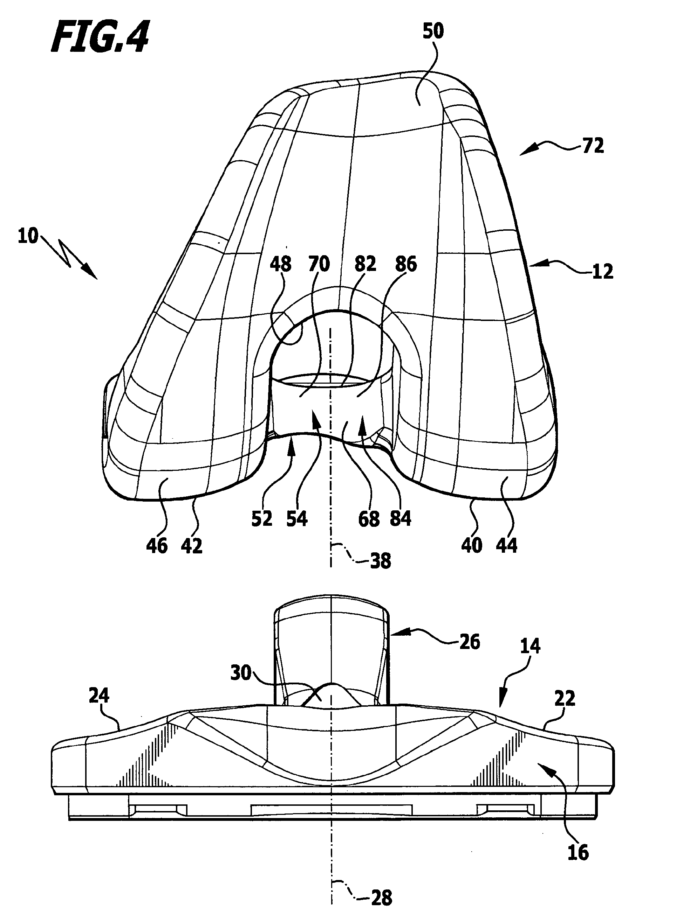

- Figur 4:: eine Ansicht der Kniegelenkendoprothese aus Figur 3 von vorne;

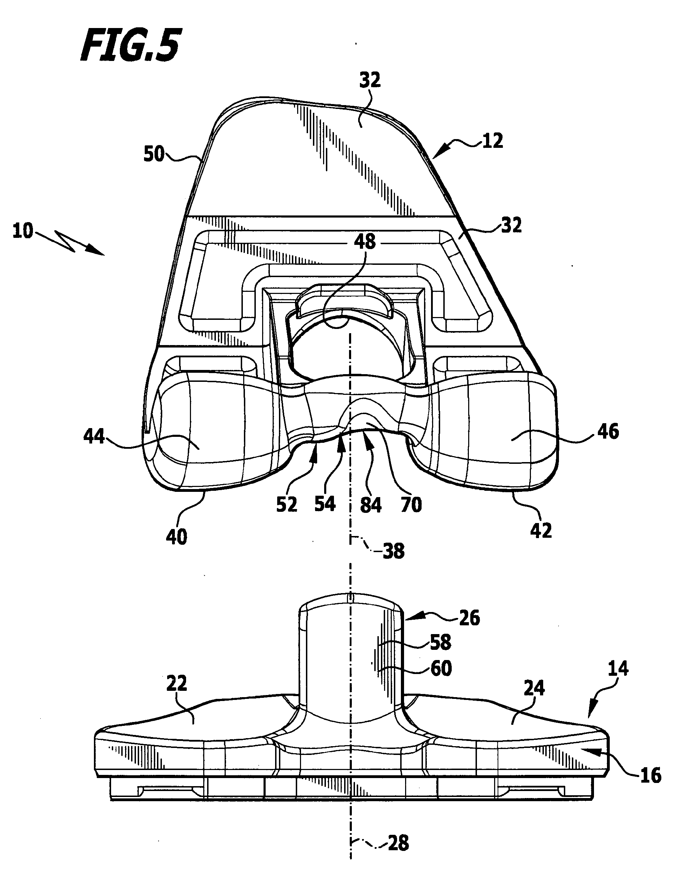

- Figur 5:: eine Ansicht der Kniegelenkendoprothese aus Figur 3 von hinten;

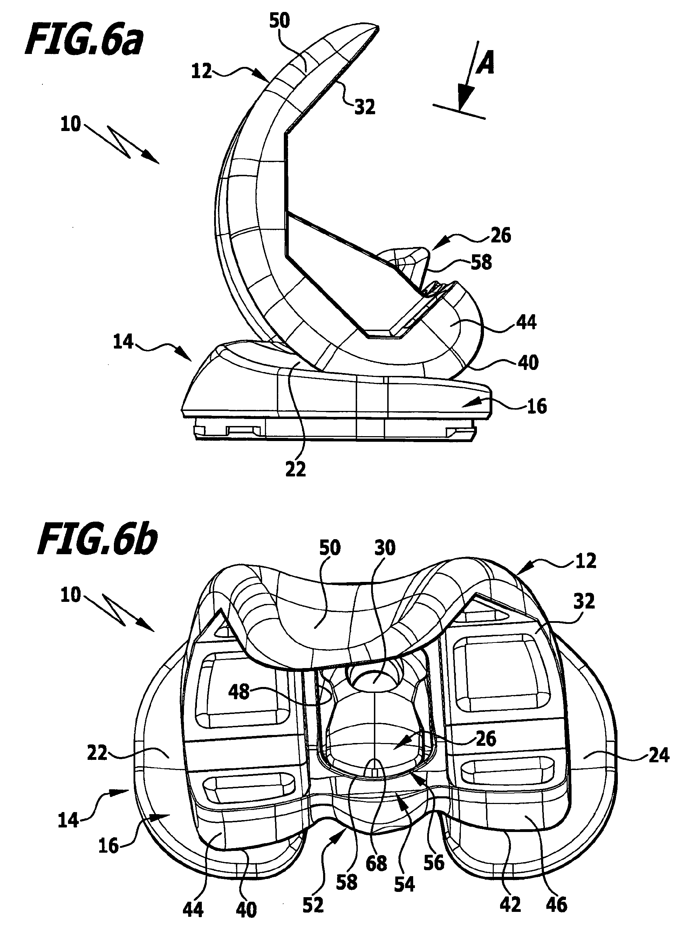

- Figur 6a:: eine Ansicht der Kniegelenkendoprothese aus Figur 2 von der Seite;
- Figur 6b:: eine Draufsicht auf die Kniegelenkendoprothese aus Figur 6a in Richtung des Pfeils A;
- Figur 7a:: eine Schnittansicht der Kniegelenkendoprothese aus Figur 6a in einer Beugestellung von etwa 45°;
- Figur 7b:: eine Schnittansicht längs Linie 7b-7b in Figur 7a;
- Figur 8a:: eine Schnittansicht der Kniegelenkendoprothese aus Figur 6a in einer Beugestellung von etwa 60°;
- Figur 8b:: eine Schnittansicht längs Linie 8b-8b in Figur 8a;
- Figur 9a:: eine Schnittansicht der Kniegelenkendoprothese aus Figur 6a in einer Beugestellung von etwa 90°;
- Figur 9b:: eine Schnittansicht längs Linie 9b-9b in Figur 9a;
- Figur 10a:: eine Schnittansicht der Kniegelenkendoprothese aus Figur 6a in einer Beugestellung von etwa 120°;
- Figur 10b:: eine Schnittansicht längs Linie 10b-10b in Figur 10a;
- Figur 11a:: eine Schnittansicht der Kniegelenkendoprothese aus Figur 6a in einer Beugestellung von etwa 135°;
- Figur 11b:: eine Schnittansicht längs Linie 11b-11b in Figur 11a;
- Figur 12:: eine schematische Darstellung einer Beugungsbewegung der Kniegelenkendoprothese aus Figur 1a in einem Flexionswinkelbereich zwischen 45 ° und 135 °; und
- Figur 13:: eine vergrößerte Ausschnittsansicht der zusammenwirkenden Führungselemente einer Rotationsführungseinrichtung der Kniegelenkendoprothese aus Figur 12.

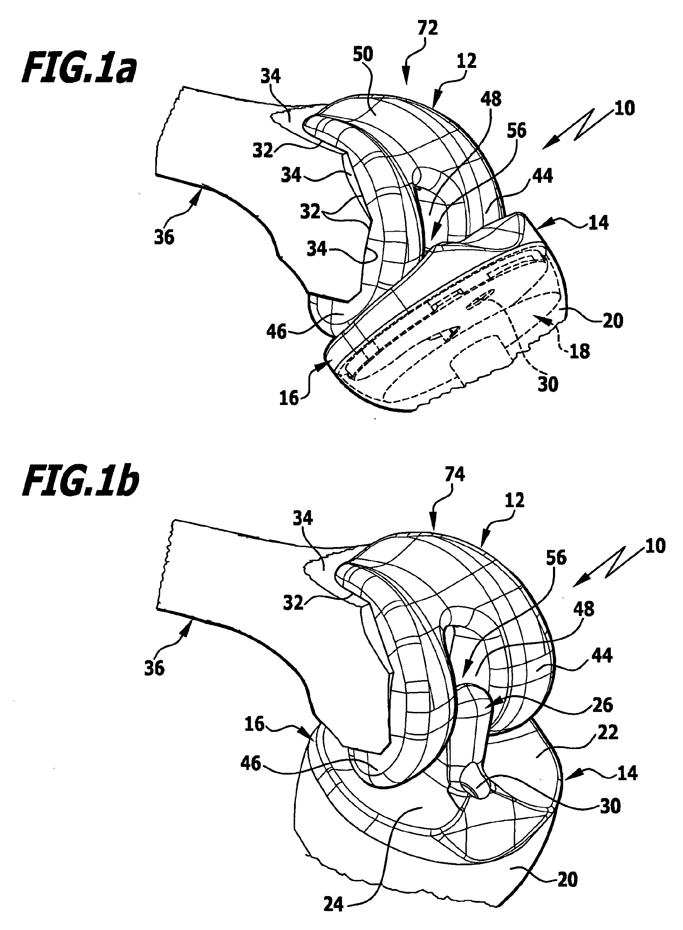

In den Figuren ist ein Ausführungsbeispiel einer insgesamt mit dem Bezugszeichen 10 bezeichneten Kniegelenkendoprothese schematisch dargestellt, welche eine Femurkomponente 12 und eine Tibiakomponente 14 umfasst. Die Tibiakomponente 14 wiederum umfasst ein einstückig ausgebildetes Meniskusteil 16 sowie ein in Figur 1 schematisch gestrichelt dargestelltes Tibiateil 18, welches an einer teilweise resezierten Tibia 20 eines Patienten verankerbar ist. Das Tibiateil 18 kann ein- oder mehrteilig ausgebildet sein, wobei zur Ausbildung eines modularen Tibiateils 18 typischerweise mehrere Teile, also mindestens zwei, vorgesehen sein können, insbesondere unterschiedlich lange Schäfte.

Das Meniskusteil 16 umfasst eine mediale Gelenkfläche 22 und eine laterale Gelenkfläche 24, die auf einer Oberseite des Meniskusteils 16 ausgebildet sind. Zwischen den Gelenkflächen 22 und 24 ist von der Oberseite des Meniskusteils in Form eines Vorsprungs abstehend ein erstes Führungselement 26 ausgebildet. Das Meniskusteil 16 ist vollständig spiegelsymmetrisch zu einer ersten Spiegelebene 28 ausgebildet, die in anterior-posteriorer Richtung verläuft. Zur Befestigung des Meniskusteils 16 am Tibiateil 18 dient eine nicht dargestellte Schraube, welche eine symmetrisch am Meniskusteil 16 ausgebildete Bohrung 30 durchgreift. Zur Aufnahme eines Kopfes der Schraube ist die Bohrung 30 in Richtung auf die Femurkomponente 12 hin einstufig erweitert. Die Bohrung 30 ist an das erste Führungselement 26 in anteriorer Richtung angrenzend angeordnet und stellt eine Verbindung zwischen Ober- und Unterseite des Meniskusteils 16 her.

Die Femurkomponente 12 umfasst eine mehrere Flächenabschnitte umfassende Knochenanlagefläche 32, die an entsprechend präparierte Schnittflächen 34 eines präparierten Femurs 36 anlegbar und insbesondere mittels Knochenzement festlegbar sind. Die Flächenabschnitte sind vorzugsweise mindestens teilweise eben und mit Ausnehmungen für den Knochenzement versehen.

In Richtung auf die Tibiakomponente 14 hin weisend sind an der Femurkomponente 12 zwei spiegelsymmetrisch zu einer zweiten Spiegelebene 38 ausgebildete Gelenkflächen vorgesehen, nämlich eine mediale Gelenkfläche 40 sowie eine laterale Gelenkfläche 42, welche einerseits eine mediale Kondyle 44 und andererseits eine laterale Kondyle 46 definieren. Die beiden Kondylen 44 und 46 sind durch eine Aussparung 48 voneinander getrennt und in einem anterioren Endbereich 50 miteinander verbunden. Die Kondylen 44 und 46 sind in einem posterioren Endbereich 52 über ein zweites Führungselement 54 miteinander verbunden. Das zweite Führungselement 54 erstreckt sich im Wesentlichen quer zur zweiten Spiegelebene 38. Es ist nicht spiegelsymmetrisch zur zweiten Spiegelebene 38 ausgebildet, wie nachfolgend noch im Einzelnen dargelegt wird.

Die medialen Gelenkflächen 22 und 40 sowie die lateralen Gelenkflächen 24 und 42 sind paarweise korrespondierend zueinander ausgebildet, um jeweils eine überlagerte Gleit-Abroll-Bewegung zwischen Femur- und Tibiakomponente 12, 14 zu ermöglichen.

Das erste Führungselement 26 und das zweite Führungselement 54 bilden jeweils einen Teil einer insgesamt mit dem Bezugszeichen 56 bezeichneten Rotationsführungseinrichtung. Das erste Führungselement 26 umfasst eine erste Führungsfläche 58, die ebenfalls spiegelsymmetrisch zur ersten Spiegelebene 28 ausgebildet ist. Sie bildet einen Ausschnitt einer äußeren Zylinderoberfläche 60. Eine Mittelpunktslinie 62 der Zylinderoberfläche 60 verläuft parallel zu einer Schnittlinie 64 zwischen der ersten Führungsfläche 58 und der ersten Spiegelebene 28. Die Schnittlinie 64 ist relativ zu einer Flächennormalen 65 einer von der Tibiakomponente 14 definierten Tibiaebene 67 geneigt. Ein erster Krümmungsradius 66 der Zylinderoberfläche 66 wird somit definiert durch einen Abstand der Mittelpunktslinie 62 und der Schnittlinie 64 voneinander. Der erste Krümmungsradius 66 liegt vorzugsweise in einem Bereich von etwa 7 mm bis etwa 15 mm und beträgt bei dem in den Figuren schematisch dargestellten Ausführungsbeispiel 12 mm.

Das zweite Führungselement 54 definiert eine zweite Führungsfläche 68, welche in einem Flexionswinkelbereich, welcher durch einen minimalen und einen maximalen Flexionswinkel definiert wird, längs eines ebenen Kreisbogenabschnitts 70 aneinander an und rollen und/oder gleiten je nach Ausgestaltung infolge einer Flexionsbewegung aneinander ab. Vorzugsweise rollen sie aneinander ab. Ein minimaler Flexionswinkel 72 beträgt bei dem in den Figuren dargestellten Ausführungsbeispiel etwa 45°, ein maximaler Flexionswinkel 74 beträgt bei dem in den Figuren dargestellten Ausführungsbeispiel 165°. Damit wird ein Flexionswinkelbereich von etwa 45° bis etwa 165° definiert. Optional kann ein minimaler Flexionswinkel in einem Bereich von etwa 20° bis etwa 60° liegen, ein maximaler Flexionswinkel in einem Bereich von etwa 120° bis etwa 190°.

Der jeweilige Flexionswinkel wird bestimmt zwischen einer Streckstellung, in welcher Femur und Tibia im Wesentlichen eine gemeinsame Längsachse definieren, und einer entsprechenden Beugestellung zwischen Femur und Tibia. Jeder Kreisbogenabschnitt 70 definiert in jeder Beugestellung somit eine Kontaktlinie zwischen der ersten Führungsfläche 58 und der zweiten Führungsfläche 68. Jeder Kreisbogenabschnitt 70 definiert ferner jeweils eine Schnittebene 76, welche jeweils senkrecht zur Schnittlinie 64 verläuft. Die Schnittlinie 64 bildet somit eine Flächennormale 78 zu allen möglichen Schnittebenen 76. In jeder Schnittebene 76 definiert die zweite Führungsfläche 68 somit einen zweiten Krümmungsradius 80, der dem ersten Krümmungsradius 66 entspricht.

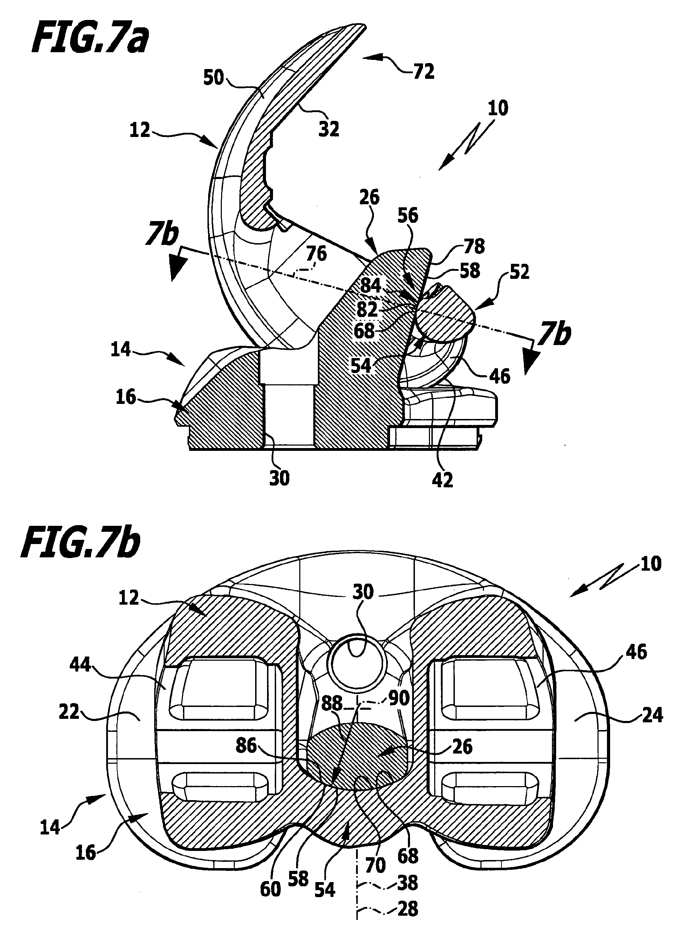

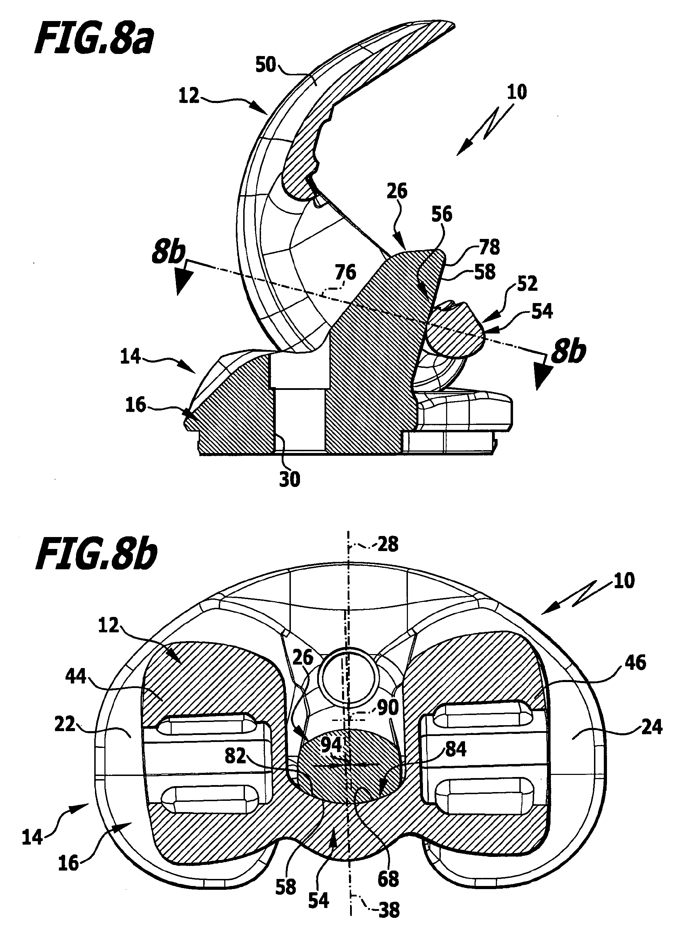

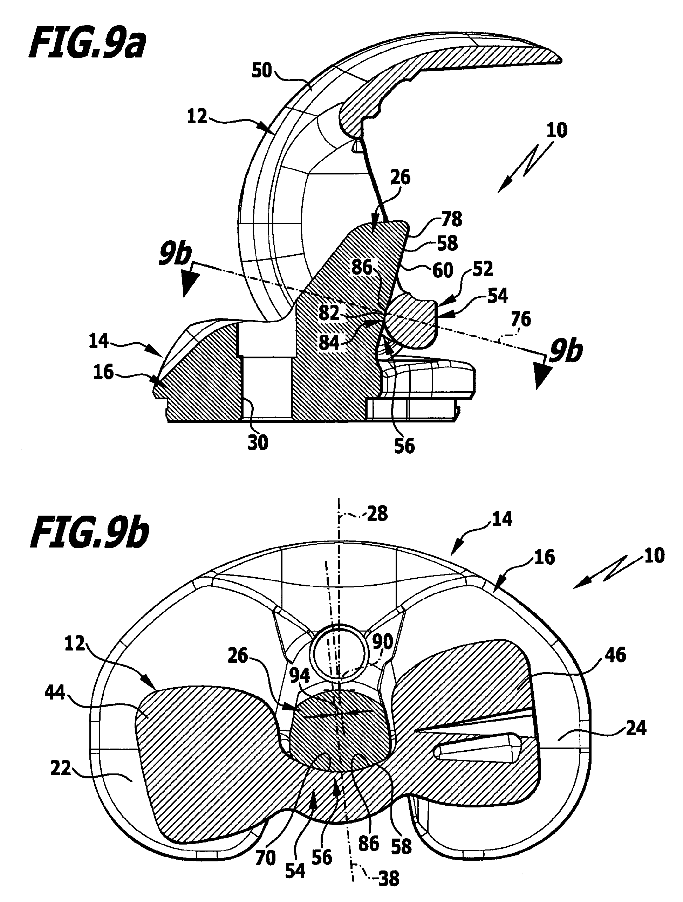

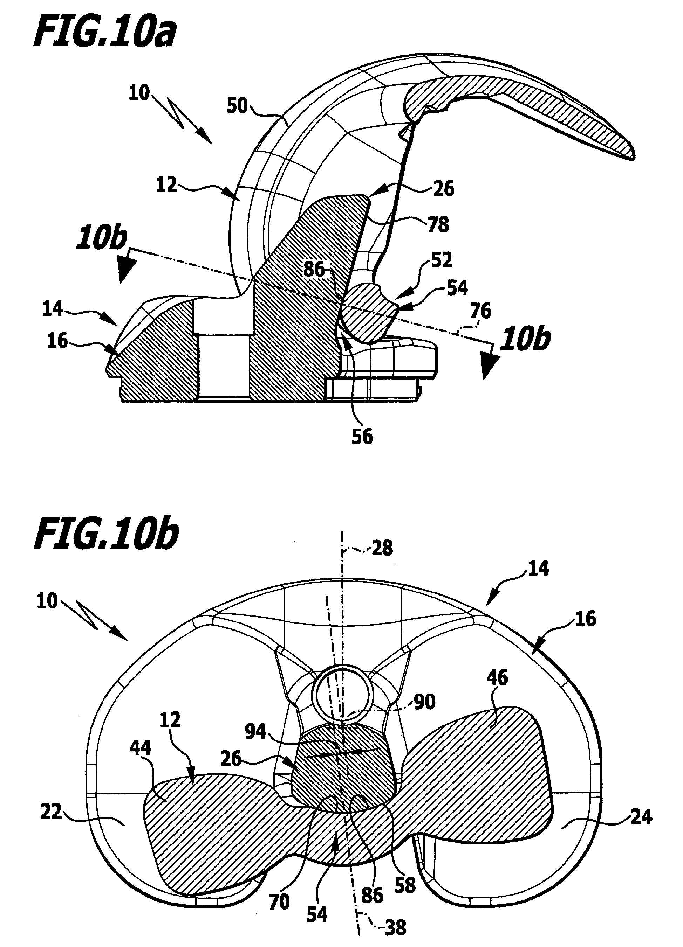

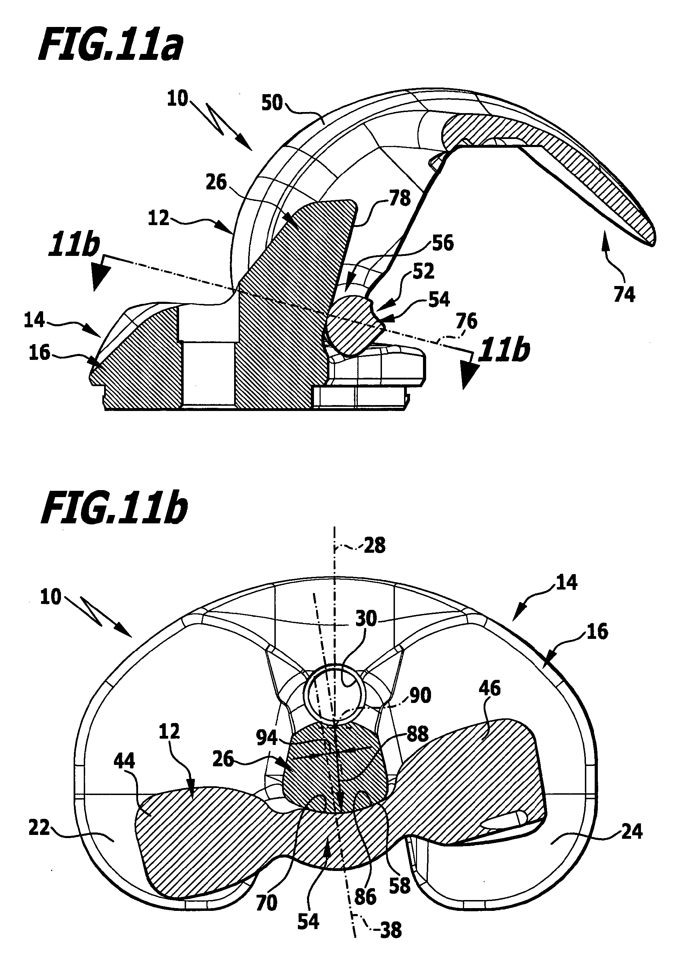

Der erste und der zweite Krümmungsradius 66, 80 sind von einem Flexionswinkel der Kniegelenkendoprothese 10 unabhängig und bleiben jeweils konstant. Dies wird erreicht durch die Zylinderoberfläche 60 in Verbindung mit der besonders ausgestalteten zweiten Führungsfläche 68. Diese wird definiert durch einen Ausschnitt einer äußeren Oberfläche 82 eines Gewindes 84 oder eines ähnlichen schraubenförmigen Körpers. Das Gewinde 84 weist einen ausgerundeten Gewindegrund 86 mit einem Gewindegrundradius 88 auf, welcher dem ersten Krümmungsradius 66 sowie dem zweiten Krümmungsradius 80 entspricht. Man kann alternativ auch sagen, dass die zweite Führungsfläche 68 einen Teil einer äußeren Schraubenfläche definiert. Das besondere der zweiten Führungsfläche 68 ist, dass der Gewindegrundradius 68 sich nicht ändert, und zwar unabhängig von einer Flexionsstellung der Femurkomponente 12 und der Tibiakomponente 14 relativ zueinander. Daher liegen diese, wie in den Figuren 7a bis 11b dargestellt und gut zu erkennen, stets längs eines ebenen Kreisbogenabschnitts 70 aneinander an.

Es sei angemerkt, dass die erste Führungsfläche 58 und die zweite Führungsfläche 68 nicht miteinander in Kontakt stehen für Flexionswinkel kleiner etwa 45°, also für Flexionswinkel, die kleiner als der minimale Flexionswinkel 72 sind. Der minimale Flexionswinkel bildet somit einen Grenzwinkel.

Aufgrund der identischen Krümmungsradien 66 und 80 der ersten und zweiten Führungsflächen 58 und 68 liegen die Mittelpunkte 90 der Kreisbogenabschnitte 70 alle auf der Mittelpunktslinie 62, die eine Gerade definiert.

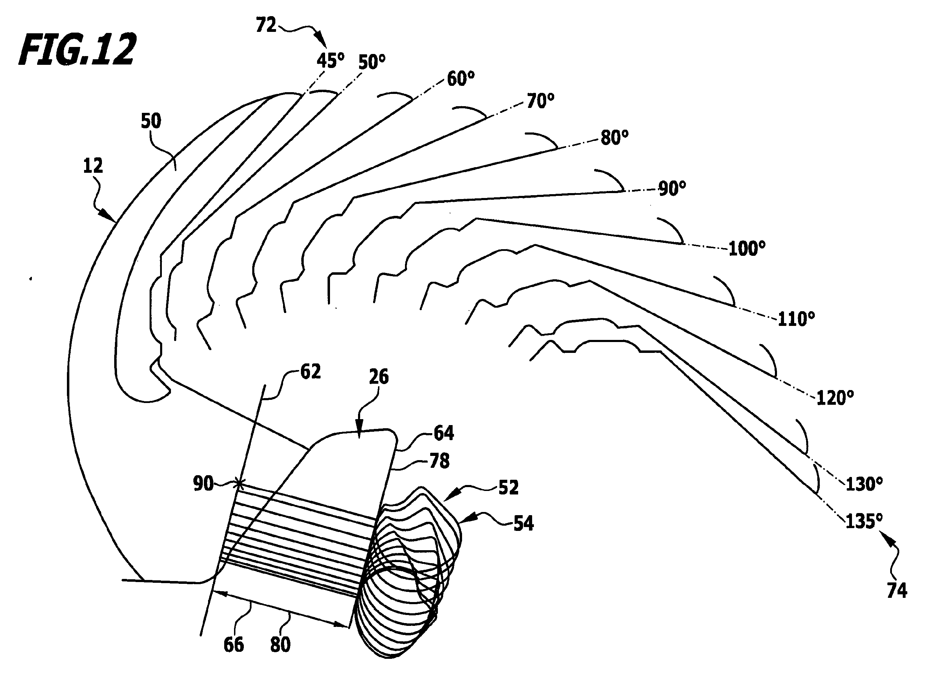

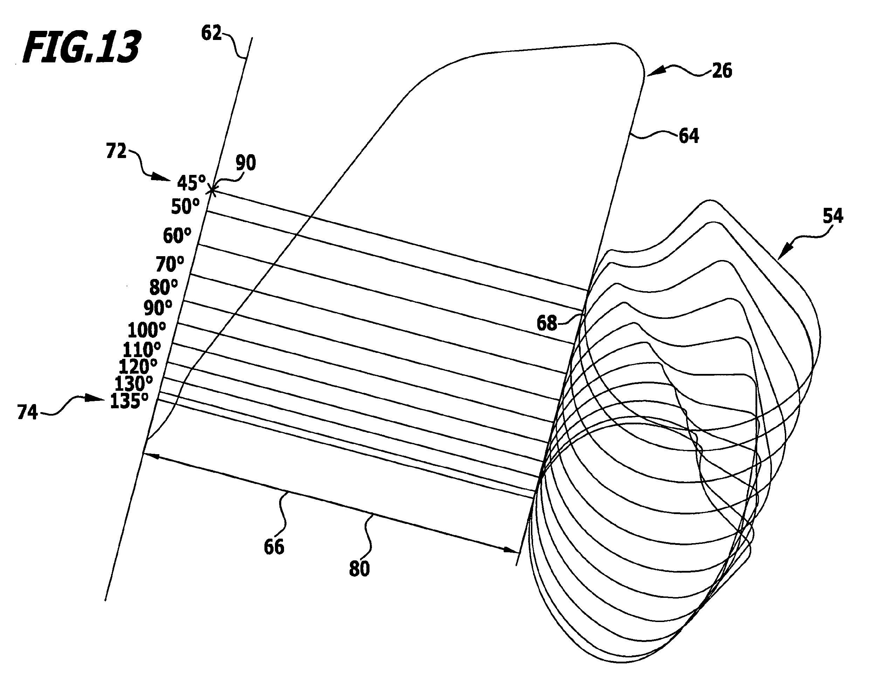

Wie in den Figuren 12 und 13 schematisch dargestellt, wandert das zweite Führungselement 54 bei einer Flexion des Knies mit zunehmendem Flexionswinkel in Richtung auf das Meniskusteil 16 hin. Bei dieser Abroll- und/oder Gleitbewegung, vorzugsweise handelt es sich um eine reine Abrollbewegung, der ersten und zweiten Führungselemente 26 und 54 relativ zueinander, wird gleichzeitig die zweite Spiegelebene 38 in medialer Richtung bezogen auf eine sämtliche Mittelpunkte 90 enthaltende Mittelpunktsebene 92 etwas versetzt. Ein Abstand 94 zwischen der zweiten Spiegelebene 38 und der Mittelpunktsebene 92 nimmt in Abhängigkeit des Flexionswinkels nichtlinear zu. Für Flexionswinkel unterhalb des minimalen Flexionswinkels 72 beträgt der Abstand 94 Null, bei einem Flexionswinkel von 90° etwa 1,4 mm und bei einem Flexionswinkel von 120° etwa 2,0 mm.

Diese Verschiebung in Querrichtung wird erreicht durch die schraubenförmige Ausgestaltung der zweiten Führungsfläche 68, welche aufgrund einer Flexionsbewegung eine Transversalbewegung bewirkt. Diese Rotations- und Seitwärtsbewegung der Femurkomponente 12 relativ zur Tibiakomponente 14 ist gut in den Figuren 7b bis 11b zu erkennen. Die Mittelpunktsebene 92 schneidet jeweils die erste Spiegelebene 28 jeweils in einem in einem Mittelpunkt 90, wie aus den in den Figuren 7b bis 11b dargestellten Schnittansichten ersichtlich. Da die Mittelpunktsebene 92 per Definition parallel zur zweiten Spiegelebene 38 verläuft, das Rotationszentrum durch die Rotationsführungseinrichtung 56 aber definiert wird durch die Mittelpunktslinie 62, ergibt sich folglich eine Verschiebung der Femurkomponente 12 und damit der zweiten Spiegelebene 38 infolge einer Beugung des Knies in medialer Richtung.

Betrachtet man also das Gesamtsystem aus Femurkomponente 12 und Tibiakomponente, sind die Mittelpunkte 90 in den zugehörigen Schnittebenen 76 jeweils deckungsgleich, was sich durch die identischen ersten und zweiten Krümmungsradien 66 und 80 zwangsläufig ergibt. Der jeweilige Mittelpunkt 90 in einer der Schnittebenen 76 liegt folglich immer auf der Schnittlinie zwischen der Mittelpunktsebene 92 und der ersten Spiegelebene 28, und zwar unabhängig vom jeweiligen Flexionswinkel.

Während der Abrollbewegung, also während eines Gangzyklus mit zunehmenden Flexionswinkel, rollt das zweite Führungselement 54 am ersten Führungselement 26 ab und vollführt, wie bereits dargelegt, gleichzeitig eine Abwärtsbewegung am ersten Führungselement 26 sowie eine Rotationsbewegung um die Mittelpunktslinie 62. Der Drehpunkt der Femurkomponente 12 liegt dabei jeweils im gemeinsamen Mittelpunkt 90 der ersten und zweiten Führungsflächen 58 und 68 und somit auf der Mittelpunktslinie 62. Die Rotationsbewegung wird bewirkt durch die Exzentrizität des Mittelpunkts 90 der zweiten Führungsfläche 68, und zwar aufgrund dessen unterschiedlicher Lage in unterschiedlichen, aber zueinander parallel Schnittebenen 76 und führt dazu, dass die Kniegelenkendoprothese 10 mit zunehmender Beugung die gewünschte und eingangs beschriebene "Medial-Pivot"-Kinematik nachbildet. Bedingt durch die Position des Drehpunktes ist die Kinematik, die die vorstehend beschriebene Kniegelenkendoprothese 10 vollführt, jedoch keine reine "Medial-Pivot"-Charakteristik. Dies bedeutet, dass die mediale Gelenkfläche 22 und die mediale Gelenkfläche 40 sich nicht stets am selben Kontaktpunkt berühren, welcher idealerweise unabhängig von einem Beugungswinkel ist. Vielmehr vollführt die Femurkomponente 12 eine Rotationsbewegung, die lateralseitig etwas größer ist als medialseitig.

## Patentansprüche

1. Kniegelenkendoprothese (10) mit einer Femurkomponente (12) und einer Tibiakomponente (14), welche zusammenwirkende und miteinander in Kontakt stehende Gelenkflächen (22, 24, 40, 42) umfassen, wobei ferner eine Rotationsführungseinrichtung (56) vorgesehen ist zum Führen einer Zwangsrotation der Femurkomponente (12) und der Tibiakomponente (14) relativ zueinander um ein mediales Rotationszentrum infolge einer Flexion der Kniegelenkendoprothese (10), welche Rotationsführungseinrichtung (56) ein erstes Führungselement (26) mit einer ersten Führungsfläche (58) und ein zweites Führungselement (54) mit einer zweiten, mit der ersten Führungsfläche (58) zusammenwirkenden Führungsfläche (68) umfasst, wobei die Tibiakomponente (14) das erste Führungselement (26) und die Femurkomponente (12) das zweite Führungselement (54) umfassen, **dadurch gekennzeichnet, dass** die erste Führungsfläche (58) einen ersten Krümmungsradius (66) definiert, dass die zweite Führungsfläche (68) einen zweiten Krümmungsradius (80) definiert und dass der erste und zweite Krümmungsradius(66, 80) identisch sind, dass die zweite Führungsfläche (68) definiert wird durch einen Ausschnitt einer äußeren Oberfläche (82) eines Gewindes (84), welches einen ausgerundeten Gewindegrund (86) mit einem Gewindegrundradius (88) aufweist, welcher dem ersten Krümmungsradius (66) entspricht.

2. Kniegelenkendoprothese nach Anspruch 1, **dadurch gekennzeichnet, dass** der erste und der zweite Krümmungsradius (66, 80) von einem Flexionswinkel der Kniegelenkendoprothese (10) unabhängig sind.

3. Kniegelenkendoprothese nach Anspruch 1 oder 2, **dadurch gekennzeichnet, dass** die erste Führungsfläche (58) spiegelsymmetrisch zu einer in anterior-posteriorer Richtung verlaufenden ersten Spiegelebene (28) ausgebildet ist.

4. Kniegelenkendoprothese nach einem der voranstehenden Ansprüche, **dadurch gekennzeichnet, dass** die erste Führungsfläche (58) einen Ausschnitt einer Zylinderoberfläche (60) definiert.

5. Kniegelenkendoprothese nach einem der voranstehenden Ansprüche, **dadurch gekennzeichnet, dass** die erste und die zweite Führungsfläche (58, 68) in einem Flexionswinkelbereich, welcher durch einen minimalen und einen maximalen Flexionswinkel (72, 74) definiert wird, längs eines ebenen Kreisbogenabschnitts (70) aneinander anliegen und bei einer Flexionsbewegung aneinander abrollen und/oder aneinander abgleiten.

6. Kniegelenkendoprothese nach einem der voranstehenden Ansprüche, **dadurch gekennzeichnet, dass** die Rotationsführungseinrichtung (56) eine Rotationsachse einer Rotationsbewegung der Femurkomponente (12) und der Tibiakomponente (14) relativ zueinander definiert und dass die Rotationsachse parallel zu einer Schnittlinie (64) zwischen der ersten Führungsfläche (58) und der ersten Spiegelebene (28) verläuft.

7. Kniegelenkendoprothese nach Anspruch 6, **dadurch gekennzeichnet, dass** der Kreisbogenabschnitt (70) eine Schnittebene (76) definiert, welche senkrecht zur Schnittlinie (64) verläuft.

8. Kniegelenkendoprothese nach einem der voranstehenden Ansprüche, **dadurch gekennzeichnet, dass** die Femurkomponente (12) zwei Gelenkflächen (40, 42) umfasst, welche spiegelsymmetrisch zu einer im Wesentlichen in anterior-posteriorer Richtung verlaufenden zweiten Spiegelebene (38) ausgebildet sind.

9. Kniegelenkendoprothese nach Anspruch 8, **dadurch gekennzeichnet, dass** die zweite Führungsfläche (68) derart ausgebildet ist, dass bei einer Flexion der Kniegelenkendoprothese (10) mit einem Flexionswinkel im Flexionswinkelbereich der Kreisbogenabschnitt (70) für jeden Flexionswinkel einen Mittelpunkt (90) definiert, und dass die Mittelpunkte (90) auf einer Mittelpunktsebene (92) liegen, welche parallel zur zweiten Spiegelebene (38) in lateraler Richtung versetzt verläuft.

10. Kniegelenkendoprothese nach Anspruch 9, **dadurch gekennzeichnet, dass** ein Abstand (94) der Mittelpunktsebene (92) von der zweiten Spiegelebene (38) in Abhängigkeit des Flexionswinkels zunimmt.

11. Kniegelenkendoprothese nach einem der Ansprüche 8 bis 10, **dadurch gekennzeichnet, dass** in einer Extensionsstellung der Kniegelenkendoprothese (10) die erste Spiegelebene (28) und die zweite Spiegelebene (38) identisch sind.

12. Kniegelenkendoprothese nach einem der voranstehenden Ansprüche, **dadurch gekennzeichnet, dass** die Tibiakomponente (14) ein Tibiateil (18) und ein am Tibiateil (18) gelagertes Meniskusteil (16) umfasst und dass das Meniskusteil (16) mit den Gelenkflächen (40, 42) der Femurkomponente (12) zusammenwirkende Gelenkflächen (22, 24) umfasst.

13. Kniegelenkendoprothese nach Anspruch 12, **dadurch gekennzeichnet, dass** das Meniskusteil (16) das erste Führungselement (26) umfasst.

14. Kniegelenkendoprothese nach Anspruch 12 oder 13, **dadurch gekennzeichnet, dass** das Meniskusteil (16) und/oder das Tibiateil (18) spiegelsymmetrisch zu einer in anterior-posteriorer Richtung verlaufenden ersten Spiegelebene (28) ausgebildet sind.

15. Kniegelenkendoprothese nach einem der voranstehenden Ansprüche, **dadurch gekennzeichnet, dass** die Tibiakomponente (14) einstückig ausgebildet ist.

## Claims

1. A knee joint endoprosthesis (10) comprising a femur component (12) and a tibia component (14), which comprise cooperating joint surfaces (22, 24, 40, 42) that are in contact with one another, wherein furthermore, there is provided a rotation guide arrangement (56) for forcing a rotational movement of the femur component (12) and the tibia component (14) relative to each other about a medial centre of rotation as a result of a flexure of the knee joint endoprosthesis (10), which rotation guide arrangement (56) comprises a first guide element (26) having a first guidance surface (58) and a second guide element (54) having a second guidance surface (68) which cooperates with the first guidance surface (58), wherein the tibia component (14) comprises the first guide element (26) and the femur component (12) comprises the second guide element (54), **characterized in that** the first guidance surface (58) defines a first radius of curvature (66), **in that** the second guidance surface (68) defines a second radius of curvature (80) and **in that** the first and second radii of curvature (66, 80) are identical, **in that** the second guidance surface (68) is defined by a section of an outer surface (82) of a thread (84) which has a rounded thread root (86) having a thread-root radius (88) which corresponds to the first radius of curvature (66).

2. A knee joint endoprosthesis in accordance with Claim 1, **characterized in that** the first and the second radii of curvature (66, 80) are independent of a flexion angle of the knee joint endoprosthesis (10).

3. A knee joint endoprosthesis in accordance with Claim 1 or 2, **characterized in that** the first guidance surface (58) is configured to be mirror-symmetrical with respect to a first mirror plane (28) running in the anterior-posterior direction.

4. A knee joint endoprosthesis in accordance with any of the preceding Claims, **characterized in that** the first guidance surface (58) defines a section of a cylinder surface (60).

5. A knee joint endoprosthesis in accordance with any of the preceding Claims, **characterized in that** the first and the second guidance surface (58, 68) abut against each other along an even circular arc section (70) within a flexion angle range which is defined by a minimum and a maximum flexion angle (72, 74), and roll upon one another and/or slide upon one another during a flexural movement.

6. A knee joint endoprosthesis in accordance with any of the preceding Claims, **characterized in that** the rotation guide arrangement (56) defines a rotational axis for the rotational movement of the femur component (12) and the tibia component (14) relative to each other, and **in that** the rotational axis runs parallel to a line of intersection (64) between the first guidance surface (58) and the first mirror plane (28).

7. A knee joint endoprosthesis in accordance with Claim 6, **characterized in that** the circular arc section (70) defines a plane of intersection (76) which runs perpendicularly to the line of intersection (64).

8. A knee joint endoprosthesis in accordance with any of the preceding Claims, **characterized in that** the femur component (12) comprises two joint surfaces (40, 42) which are configured to be mirror-symmetrical with respect to a second mirror plane (38) running substantially in the anterior-posterior direction.

9. A knee joint endoprosthesis in accordance with Claim 8, **characterized in that** the second guidance surface (68) is configured such that a centre point (90) is defined for each flexion angle when there is flexure of the knee joint endoprosthesis (10) with a flexion angle within the flexion angle range of the circular arc section (70), and **in that** the centre points (90) lie on a centre point plane (92) which runs parallel to the second mirror plane (38) and is offset in the lateral direction.

10. A knee joint endoprosthesis in accordance with Claim 9, **characterized in that** a spacing (94) of the centre point plane (92) from the second mirror plane (38) increases in dependence on the flexion angle.

11. A knee joint endoprosthesis in accordance with any of the Claims 8 to 10, **characterized in that** the first mirror plane (28) and the second mirror plane (38) are identical in an extended position of the knee joint endoprosthesis (10).

12. A knee joint endoprosthesis in accordance with any of the preceding Claims, **characterized in that** the tibia component (14) comprises a tibial part (18) and a meniscus part (16) which is mounted on the tibial part (18) and **in that** the meniscus part (16) comprises joint surfaces (22, 24) which cooperate with the joint surfaces (40, 42) of the femur component (12).

13. A knee joint endoprosthesis in accordance with Claim 12, **characterized in that** the meniscus part (16) comprises the first guide element (26).

14. A knee joint endoprosthesis in accordance with Claim 12 or 13, **characterized in that** the meniscus part (16) and/or the tibial part (18) are configured to be mirror-symmetrical with respect to a first mirror plane (28) running in the anterior-posterior direction.

15. A knee joint endoprosthesis in accordance with any of the preceding Claims, **characterized in that** the tibia component (14) is constructed in one piece manner.

## Revendications

1. Endoprothèse d'articulation du genou (10) avec un composant fémur (12) et un composant tibia (14), qui comprennent des surfaces d'articulation (22, 24, 40, 42) qui coopèrent et qui sont en contact entre elles, où en outre il est prévu un dispositif de guidage en rotation (56) pour le guidage d'une rotation commandée du composant fémur (12) et du composant tibia (14) l'un par rapport à l'autre autour d'un centre de rotation médian par suite d'une flexion de l'endoprothèse d'articulation du genou (10), lequel dispositif de guidage en rotation (56) comprend un premier élément de guidage (26) avec une première surface de guidage (58) et un deuxième élément de guidage (54) avec une deuxième surface de guidage (68) qui coopère avec la première surface de guidage (58), où le composant tibia (14) comprend le premier élément de guidage (26) et le composant fémur (12) comprend le deuxième élément de guidage (54), **caractérisée en ce que** la première surface de guidage (58) définit un premier rayon de courbure (66), **en ce que** la deuxième surface de guidage (68) définit un deuxième rayon de courbure (80) et **en ce que** le premier et le deuxième rayon de courbure (66, 80) sont identiques, **en ce que** la deuxième surface de guidage (68) est définie par une tranche d'une surface externe (82) d'un filet (84), qui comporte un fond de filet (86) arrondi avec un rayon de fond de filet (88) qui correspond au premier rayon de courbure (66).

2. Endoprothèse d'articulation du genou selon la revendication 1, **caractérisée en ce que** le premier et le deuxième rayon de courbure (66, 80) sont indépendants d'un angle de flexion de l'endoprothèse d'articulation du genou (10).

3. Endoprothèse d'articulation du genou selon la revendication 1 ou 2, **caractérisée en ce que** la première surface de guidage (58) est agencée symétriquement dans un miroir par rapport à un premier plan de miroir (28) qui s'étend en direction antéro-postérieure.

4. Endoprothèse d'articulation du genou selon l'une des revendications précédentes, **caractérisée en ce que** la première surface de guidage (58) définit une tranche d'une surface cylindrique (60).

5. Endoprothèse d'articulation du genou selon l'une des revendications précédentes, **caractérisée en ce que** la première et la deuxième surface de guidage (58, 68) sont situées côte à côte dans un domaine d'angle de flexion qui est défini par un angle de flexion minimal et un angle de flexion maximal (72, 74) le long d'une section d'arc de cercle plane (70) et roulent l'une sur l'autre et/ou glissent l'une sur l'autre lors d'un mouvement de flexion.

6. Endoprothèse d'articulation du genou selon l'une des revendications précédentes, **caractérisée en ce que** le dispositif de guidage en rotation (56) défini un axe de rotation d'un mouvement de rotation du composant fémur (12) et du composant tibia (14) l'un par rapport à l'autre et **en ce que** l'axe de rotation s'étend parallèlement à une ligne d'intersection (64) entre la première surface de guidage (58) et le premier plan de miroir (28).

7. Endoprothèse d'articulation du genou selon la revendication 6, **caractérisée en ce que** la section d'arc de cercle (70) définit un plan d'intersection (76) qui s'étend perpendiculairement à la ligne d'intersection (64).

8. Endoprothèse d'articulation du genou selon l'une des revendications précédentes, **caractérisée en ce que** le composant fémur (12) comprend deux surfaces d'articulation (40, 42) qui sont disposées de manière symétrique dans un miroir par rapport à un deuxième plan de miroir (38) qui s'étend sensiblement en direction antéro-postérieure.

9. Endoprothèse d'articulation du genou selon la revendication 8, **caractérisée en ce que** la deuxième surface de guidage (68) est agencée de telle manière que, lors d'une flexion de l'endoprothèse d'articulation du genou (10) avec un angle de flexion dans le domaine d'angle de flexion, la section d'arc de cercle (70) définit un point central (90) pour chaque angle de flexion, et **en ce que** les points centraux (90) sont situés sur un plan de points centraux (92) qui s'étend parallèlement au deuxième plan de miroir (38) décalé en direction latérale.

10. Endoprothèse d'articulation du genou selon la revendication 9, **caractérisée en ce qu'**une distance (94) du plan de points centraux (92) avec le deuxième plan de miroir (38) augmente en fonction de l'angle de flexion.

11. Endoprothèse d'articulation du genou selon l'une des revendications 8 à 10, **caractérisée en ce que**, dans une position d'extension de l'endoprothèse d'articulation du genou (10), le premier plan de miroir (28) et le deuxième plan de miroir (38) sont identiques.

12. Endoprothèse d'articulation du genou selon l'une des revendications précédentes, **caractérisée en ce que** le composant tibia (14) comprend une partie tibia (18) et une partie ménisque (16) montée sur la partie tibia (18) et **en ce que** la partie ménisque (16) comprend des surfaces d'articulation (22, 24) coopérant avec les surfaces d'articulation (40, 42) du composant fémur (12).

13. Endoprothèse d'articulation du genou selon la revendication 12, **caractérisée en ce que** la partie ménisque (16) comprend le premier élément de guidage (26).

14. Endoprothèse d'articulation du genou selon la revendication 12 ou 13, **caractérisée en ce que** la partie ménisque (16) et/ou la partie tibia (18) sont agencées de manière symétrique dans un miroir par rapport à un premier plan de miroir (28) qui s'étend en direction antéro-postérieure.

15. Endoprothèse d'articulation du genou selon l'une des revendications précédentes, **caractérisée en ce que** le composant tibia (14) est agencé d'une pièce.
